# EUROPEAN PATENT APPLICATION

(11) **EP 3 771 719 A1**
(43) Date of publication of application: **03.02.2021**
(21) Application number: 19189787.5
(22) Date of filing: 02.08.2019
(51) Int. Cl.: C07K 7/00, A61K 38/00

(54) **A PEPTIDE BINDING TO TAU-PROTEIN**

(71) Applicant: Hochschule für angewandte Wissenschaften Fachhochschule Coburg, 96450 Coburg (DE)
(72) Inventor: Funke, Susanne Aileen, 96242 Sonnefeld (DE); Malhis, Marwa, 96450 Coburg (DE)
(74) Representative: Engelhard, Markus

(57) **Abstract**

The present invention relates to peptides binding to tau-protein, and to a pharmaceutical composition comprising such peptide(s). Furthermore, the present invention relates to uses of such peptide(s) and pharmaceutical composition. Moreover, the present invention relates to a kit comprising such peptide.

SEQUENCE LISTING

<110> Hochschule für angewandte Wissenschaften Coburg

<120> A peptide binding to tau-protein

<130> H33320EP

<160> 8

<170> BiSSAP 1.3.2

<210> 1
<211> 12
<212> PRT
<213> Artificial Sequence

<223> "L-amino acid residues or D-amino acid residues or combinations
thereof"

<220>
<223> L-amino acid residues or D-amino acid residues or combinations
      thereof

<400> 1
Asp Pro Leu Lys Ala Arg His Thr Ser Val Trp Tyr
1               5                   10

<210> 2
<211> 12
<212> PRT
<213> Artificial Sequence

<223> "retro-inverse L-enantiomer or D-enantiomer"

<220>
<223> retro-inverse L-enantiomer or D-enantiomer

<400> 2
Tyr Trp Val Ser Thr His Arg Ala Lys Leu Pro Asp
1               5                   10

<210> 3
<211> 12
<212> PRT
<213> Artificial Sequence

<223> "D-enantiomer"

<220>
<223> D-enantiomer

<400> 3
Asp Pro Leu Lys Ala Arg His Thr Ser Val Trp Tyr
1               5                   10

<210> 4
<211> 12
<212> PRT
<213> Artificial Sequence

<223> "retro-inverse D-enantiomer"

<220>
<223> retro-inverse D-enantiomer

<400> 4
Tyr Trp Val Ser Thr His Arg Ala Lys Leu Pro Asp
1               5                   10

<210> 5
<211> 6
<212> PRT
<213> Artificial Sequence

<223> "sequence stretch in PHF6*-fibrils"

<220>
<223> sequence stretch in PHF6*-fibrils

<400> 5
Val Gln Ile Ile Asn Lys
1               5

<210> 6
<211> 12
<212> PRT
<213> Artificial Sequence

<223> "L-amino acid residues or D-amino acid residues or mixtures thereof"

<220>
<223> L-amino acid residues or D-amino acid residues or mixtures
      thereof

<400> 6
Leu Thr Pro His Lys His His Lys His Leu His Ala
1               5                   10

<210> 7
<211> 12
<212> PRT
<213> Artificial Sequence

<223> "D-enantiomer"

<220>
<223> D-enantiomer

<400> 7
Leu Thr Pro His Lys His His Lys His Leu His Ala
1               5                   10

<210> 8
<211> 12
<212> PRT
<213> Artificial Sequence

<223> "retro-inverse D-enantiomer"

<220>
<223> retro-inverse D-enantiomer

<400> 8
Ala His Leu His Lys His His Lys His Pro Thr Leu
1               5                   10

## Description

The present invention relates to peptides binding to tau-protein, and to a pharmaceutical composition comprising such peptide(s). Furthermore, the present invention relates to uses of such peptide(s) and pharmaceutical composition. Moreover, the present invention relates to a kit comprising such peptide.

Alzheimer's disease is characterized by a pathological aggregation of two proteins, namely amyloid-beta-peptide (Aβ) and tau-protein. Both types of protein aggregates are considered neurotoxic, whereas the respective monomers are not toxic and have important cellular functions. One function of the Tau-protein is to bind to cytoskeletal proteins (microtubuli) and to control their composition. Neurodegenerative diseases involving deposits of tau-protein are also referred to as tauopathies. The best known tauopathy is Alzheimer's disease, however, there are also pathological tau-aggregates involved in other diseases, such as frontotemporal dementia and Parkinson's disease.

In the human central nervous system, there are different isoforms of tau-protein. The individual tau-proteins may form oligomers, which are described as cytotoxic and which can spread from cell to cell, and anormal paired helical filaments (PHF). As a consequence of these changes, pathological deposits of paired helical filaments may be observed as fibrils in the brains of patients having Alzheimer's disease.

Small molecules intended to prevent the aggregation of tau-protein have already been described. However, the major focus of present research efforts is still more on a prevention of the aggregation of Aβ. However, a reduction of plaques by an anti-Aβ-immunotherapy did not lead to an improvement of the cognitive performance of the patients thus treated. Therefore, inhibitors or modulators of tau-protein aggregation may represent a suitable supplement for therapies against Alzheimer's disease and other tauopathies.

Thus, there is a need in the art for providing new methods and compounds for the treatment of tauopathies in particular of Alzheimer's disease. More specifically, it was an object of the present invention to provide compounds that would bind to tau-protein. It is furthermore an object of the invention to provide inhibitors and/or modulators of aggregation of tau-protein.

In a first aspect, the present invention relates to a peptide binding to tau-protein, or tau-protein fragments, said peptide comprising an amino acid sequence selected from
DPLKARHTSVWY (SEQ ID NO: 1), and
YWVSTHRAKLPD (SEQ ID NO: 2) and dimers or multimers of peptides comprising such sequence(s), and homologs, derivates or fragments of peptides comprising such sequence(s).

In one embodiment, the peptide consists of an amino acid selected from
DPLKARHTSVWY (SEQ ID NO: 1), and
YWVSTHRAKLPD (SEQ ID NO: 2).

In one embodiment, said amino acid sequence is made up of L-amino acid residues or D-amino acid residues, or a combination thereof.

In one embodiment, said amino acid sequence is selected from
DPLKARHTSVWY (SEQ ID NO: 1),
YWVSTHRAKLPD (SEQ ID NO: 2),
dplkarhtsvwy (SEQ ID NO: 3), and
ywvsthraklpd (SEQ ID NO: 4),
wherein upper case letters represent L-amino acid residues or D-amino acid residues, preferably L-amino acid residues, and lower case letters represent D-amino acid residues.

In one embodiment, said peptide comprises a tag attached to said peptide to facilitate detection or purification of the peptide, or to increase solubility thereof, or to increase blood-brain-barrier permeability; or further comprising a tag attached that is directed at A-beta-protein.

In one embodiment, said peptide binds to tau-monomers, -oligomers or -fibrils , in particular to tau-oligomers or tau-fibrils comprising a peptide sequence segment having the sequence VQIINK (SEQ ID NO: 5).

In one embodiment, said peptide inhibits aggregation of monomeric tau-protein and/or aggregation or extension of tau-oligomers or tau-fibrils.

In a further aspect, the present invention relates to a pharmaceutical composition comprising a peptide according to the present invention and a pharmaceutically acceptable excipient.

In a further aspect, the present invention relates to a peptide or the pharmaceutical composition according to the present invention, for use in method of treating and/or preventing a tauopathy, wherein preferably said tauopathy is selected from Alzheimer's disease (AD), primary age-related tauopathy, frontotemporal dementia, chronic traumatic encephalopathy (CTE), progressive supranuclear palsy, corticobasal degeneration, Lytico-bodig disease, ganglioglioma, gangliocytoma, Meningioangiomatosis, postencephalitic parkinsonism, and subacute sclerosing panencephalitis.

In one embodiment, said method of treating and/or preventing comprises administering an effective amount of said peptide or of said composition to a patient in need thereof.

In a further aspect, the present invention relates to a peptide or the pharmaceutical composition according to the present invention, for use in a method of diagnosing a tauopathy, wherein preferably said tauopathy is selected from Alzheimer's disease (AD), primary age-related tauopathy, frontotemporal dementia, chronic traumatic encephalopathy (CTE), progressive supranuclear palsy, corticobasal degeneration, Lytico-bodig disease, ganglioglioma, gangliocytoma, Meningioangiomatosis, postencephalitic parkinsonism, and subacute sclerosing panencephalitis.

In one embodiment, said method of diagnosing comprises administering an effective amount of said peptide or of said composition to a subject to be tested for a tauopathy. In one embodiment, said peptide is linked to or administered together with a suitable reporter molecule that allows detection of monomeric tau-protein, tau-oligomers or of tau-fibrils and/or aggregates thereof, by a suitable detection methodology, such as positron emission tomography (PET), nuclear magnetic resonance (NMR), magnetic resonance imaging (MRI) and PET-MRI, and wherein said subject, after administration of said peptide or of said pharmaceutical composition, is subjected to said suitable detection methodology, such as PET, NMR, MRI, PET-MRI.

In a further aspect, the present invention relates to a kit for the in-vitro or in-vivo detection of tau-protein monomers, oligomers, aggregates or tau-fibrils, or for the diagnosis of a tauopathy, said kit comprising the peptide according to the present invention, in a freeze-dried form in a suitable container, a buffered solvent in a separate container for reconstitution of said peptide in solution, and, optionally, means to dispense said peptide once reconstituted in solution, such as a syringe or pipette.

In a further aspect, the present invention relates to the use of the peptide according to the present invention, in an in-vitro assay, preferably in an in-vitro immunochemistry assay, such as an enzyme-linked immunosorbent assay (ELISA) or a radio-immuno assay (RIA).

The inventors have surprisingly found that peptides according to the present invention are capable of binding to monomeric tau-protein, and aggregates of tau-protein, in particular tau-fibrils. Furthermore, they are capable of preventing the formation and/or elongation/extension of tau oligomers or -fibrils.

In a further aspect, the present invention relates to the use of a peptide according to the present invention as defined above, for the manufacture of a medicament for the prevention, treatment or diagnosis of a tauopathy, as defined above, in particular of Alzheimer's disease.

In yet a further aspect, the present invention also relates to a method of prevention, treatment or diagnosis of a tauopathy, in particular of Alzheimer's disease, wherein said method comprises administering an effective amount of said peptide or of said composition according to the present invention, as defined further above, to a patient in need thereof or to a subject to be tested.

The present inventors have provided peptides which function as inhibitors and/or modulators of the aggregation of tau-protein, and which therefore have manifold applications. Without wishing to be bound to any mechanism or any theory, the present inventors believe that the peptides in accordance with embodiments of the invention bind to tau-protein, in particular to monomeric tau-protein, tau-protein-oligomers and/or tau-fibrils, and thereby inhibit the aggregation of tau-protein, in particular of monomeric tau-protein, and/or the aggregation or extension of tau-oligomers or tau-fibrils. Also, without wishing to be bound by any theory or mechanism, the present invention further believe that the peptides in accordance with embodiments of the invention may act to modify tau-oligomeric species and tau-fibril species in such a manner that these are converted from toxic, i. e. pathologic, forms into non-toxic amorphous aggregates.

In the present application, use is made of the one-letter-code for amino acid residues and the three-letter-code for amino acid residues. Hence, amino acid residues are designated herein by reference to their respective one-letter-code or three-letter-code. Accordingly, alanine is A or Ala; arginine is R or Arg; asparagine is N or Asn; aspartic acid is D or Asp; cysteine is C or Cys; glutamine is Q or Gln; glutamate is E or Glu; glycine is G or Gly; histidine is H or His; isoleucine is I or Ile; leucine is L or Leu; lysine is K or Lys; methionine is M or Met; phenylalanine is F or Phe; proline is P or Pro; serine is S or Ser; threonine is T or Thr; tryptophan is W or Trp; tyrosine is Y or Tyr; valine is V or Val.

Sometimes, in this application, reference to amino acid sequences is made by reciting the individual residues. Where such amino acid sequence is indicated by using upper case letters only, this means that these amino acids are unspecified in terms of their chirality, i.e. the residues may be L-amino acids or D- amino acids or a mixture of the two possibilities, i.e. some of the residues in the sequence may be L-amino acids and others may be D- amino acids. In one preferred embodiment, the upper case amino acids may be all L-amino acids.

Moreover, sometimes, in this application, reference to amino acid sequences is made by reciting the individual residues as free amino acids, such as "glycine", "glutamic acid", etc., notwithstanding the fact that these residues appear in the respective amino acid sequence in their respective covalently linked form, i.e. with the individual residues linked by appropriate peptide bonds, i.e. amide bonds, between them.

In one embodiment, the N-terminus and/or the C-terminus of the peptides according to the present invention are protected. This may even be the case when the respective peptide "consists of' the respective amino acid sequence. Suitable protecting groups are manifold and are known to a person skilled in the art. For example, the N-terminus may be acetylated or formylated, or there may be an even longer chain attached such as palmitoyl. The C-terminus could be protected via formation of an amide or carbonic acid ester.

In one embodiment, the C-termini of the peptide(s) according to the present invention, are protected by an amide. In other embodiments, the C-terminus is in its free carboxy-form; in yet other embodiments, it is in esterified form. In particularly preferred embodiments, the C-termini of the peptide(s) according to the present invention, are protected by an amide, and the respective N-termini of the peptides are unprotected, i.e. in their NH₂-form (or NH₃⁺-form).

Sometimes, in this application, reference is made to "oligomers" of tau-protein or "tau-oligomers". Such a term is meant to refer to an aggregated species of tau-protein that is composed of tau-protein monomers, but that remains soluble in aqueous solution. Also, such term "oligomer" is meant to refer to different such soluble aggregated species of different sizes (i.e. different oligomers), as long as these remain soluble in aqueous solution. In contrast thereto, reference is sometimes made herein to "fibrils" of tau-protein or "tau-fibrils", which are also composed of tau-protein monomers, but are larger fibrous aggregates, which no longer stay in aqueous solution and can, for example be centrifuged and separated as insoluble aggregates. Both terms, i.e. "oligomers" and "fibrils" of tau-protein, are herein also sometimes collectively referred to as "aggregates" of tau-protein or "tau-aggregates".

In accordance with embodiments of the present invention, a peptide according to the present invention may also comprise a "tag". Such "tag" typically is attached to the peptide. Such tag may serve the purpose of facilitating detection or purification of the peptide according to the present invention to which it is attached, or it may serve the purpose of increasing solubility or blood-brain-barrier permeability. Alternatively, such tag may also serve the purpose of targeting the peptide in accordance with the present invention to a particular entity or location or to a particular other molecule. For example, such tag may itself be an entity, preferably a peptide, that binds to the Aβ-protein, which itself is believed to be involved in the pathology of Alzheimer's disease. In preferred embodiments of the present invention, such tag is a peptide itself (including a simple amino acid residue, or a dipeptide, tripeptide or other short peptide) or may be parts of another protein. As an example, a tag for facilitating the purification of a peptide may be a poly-histidine-tag. An example of a tag that facilitates detection of the peptide to which such tag is attached may be a tag composed of green fluorescent protein or derivatives thereof (GFP-tag) or may be a tag composed of a fluorescent dye or including such dye. A person skilled in the art knows about such different tags and is a able to attach such tag to peptides according to the present invention.

In accordance with some embodiments of the present invention, the peptides according to the present invention may also form dimers or multimers themselves, for example by forming disulfide bridges or by being linked to each other through other parts of the peptide(s) that are outside of SEQ ID NO: 1-4.

Sometimes reference is made herein to "homologs" or "derivates". These terms are meant to refer to variants of peptides according to the present invention which retain their capability of binding to tau-protein or tau-protein fragments, which variant peptides are at least 80% identical, preferably at least 85% identical, more preferably at least 90% identical, even more preferably at least 95% identical, and yet more preferably at least 99% identical with a peptide having an amino acid sequence selected from SEQ ID NO: 1 - SEQ ID NO: 4.

The term "treatment" or "treating", as used herein, encompasses both prophylactic treatment and therapeutic treatment. In a preferred embodiment, it specifically refers to therapeutic treatment.

The present inventors have managed to identify and select peptides that are capable of binding to tau-protein. In particular, the present inventors performed two independent phage display selections, one against monomeric tau-protein, and the other against fibrils comprising the sequence VQIINK (which specific fibrils are herein also sometimes referred to as "PHF6*"). Interestingly, in both independent selections, the sequences referred to herein sometimes as "MM3" and "MMD3" were identified, thus lending support to the fact that such sequences, as well as homologs and derivatives thereof, have a genuine binding capability. It should be noted that the present inventors also envisage variations of these sequences, wherein, for example, additional amino acid residues or amino acid residue stretches are attached which may, optionally, be further labeled. An example of such a particular tag that is envisaged is -lysine-5/6-carboxy-fluorescein (herein sometimes also abbreviated as "Lys(FAM)-NH2"). Other tags may be equally employed, and such accordingly modified sequences also form part of the present invention. Without wishing to be bound by any theory, the present inventors believe that the peptides according to the present invention inhibit aggregation of tau-protein, by their binding thereto.

Preferred sequences of the present invention are shown in table 1.

**Table 1:**

| **Amino acid sequence** | **SEQ ID NO:** | **also herein sometimes referred to as** |
|---|---|---|
| DPLKARHTSVWY | 1 | MM3 |
| | | (as L-enantiomer) |
| YWVSTHRAKLPD | 2 | MML3rev |
| | | (as retro-inverse L-enantiomer) |
| dplkarhtsvwy | 3 | MMD3 |
| | | (as D-enantiomer) |
| ywvsthraklpd | 4 | MMD3rev |
| | | (as retro-inverse D-enantiomer) |

Other sequences not forming part of the present invention, but nevertheless sometimes referred to herein are shown in table 2.

**Table 2:**

| **Amino acid sequence** | **SEQ ID NO:** | **also herein sometimes referred to as** |
|---|---|---|
| LTPHKHHKHLHA | 6 | MM2 |
| | | (as L-enantiomer) |
| ltphkhhkhlha | 7 | MMD2 |
| | | (as D-enantiomer) |
| ahlhkhhkhptl | 8 | MMD2rev |
| | | (as retro-inverse D-enantiomer) |

Furthermore, reference is made to the figures wherein
Figure 1 shows binding of peptides MM2-Lys(FAM)-NH₂ and MM3-Lys(FAM)-NH₂ to monomeric tau-protein and tau-fibrils.
Figures 2 and 3 show the results of a ThT aggregation assay of tau-protein for quantification of the relative content of fibrils in the presence of different peptides (+ heparin) that had been selected against monomeric tau-protein.
Figure 4 shows results of binding of peptide MMD3-Lys(FAM)-NH₂ to PHF6*-fibrils; and
Figure 5 shows the inhibition of aggregation of PHF6*-fibrils in the presence of peptide MMD3 and MMD3rev.

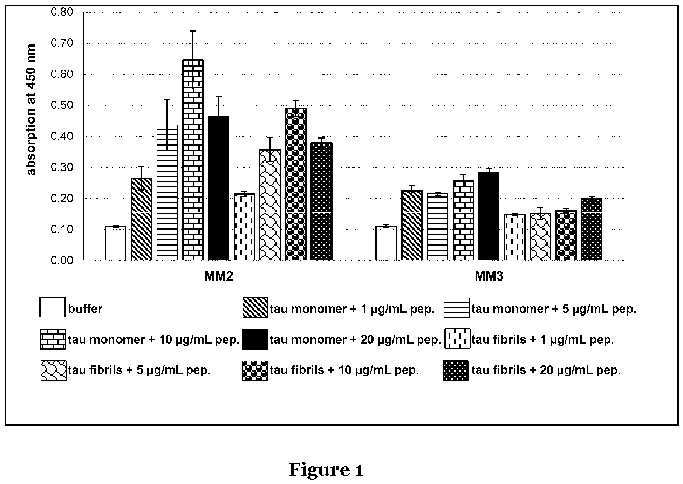

More specifically, figure 1 shows the following:
ELISA was performed to demonstrate binding of peptides to the monomeric tau-protein and to tau-fibrils. Peptides were used that had been labeled with a FAM (=5/6-carboxyfluorescein) at the C-terminus. The peptides used were MM2 (= LTPHKHHKHLHA as L-enantiomer = SEQ ID NO: 6) and MM3 (SEQ ID NO: 1), to which a Lys (FAM)-NH2-residue had been attached at the C-terminus, respectively. The wells of a 96-well plate were coated with 20 µg/ml monomeric tau-protein or tau-fibrils and were incubated for 1h. After washing three times with PBS-T (phosphate-buffered saline with 0.1 % tween), the free binding sites were blocked for over a period of 1 hour. The respective peptides MM2-Lys(FAM)-NH2 and MM3-Lys(FAM)-NH2 were added (1 µg/ml, 5 µg/ml, 10 µg/ml, 20 µg/ml) and incubated for 1h. A specific anti-FITC-HRP antibody (anti-fluorescein-isothiocyanate-horseradish-peroxidase) was added in 1:1000 dilution and was incubated for 1h. Thereafter, TMB-substrate was added and incubated for 20 min. After determination of enzyme reaction by addition of 20% sulfuric acid, the absorption at 450 nm was determined. Measurements were performed in triplicate and the average values of the measured absorption of these triplicate samples as well as their standard deviations are shown in figure 1.

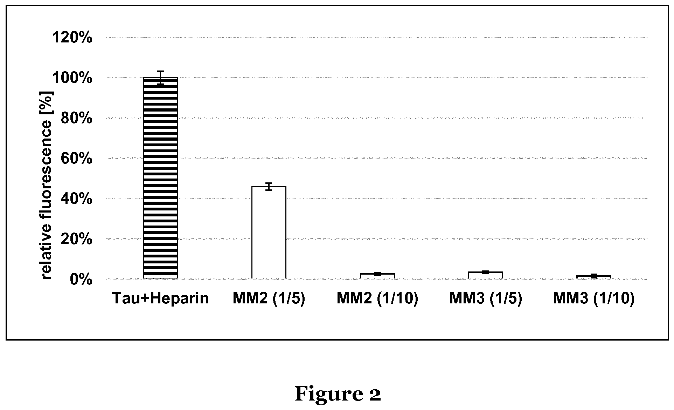

### Figure 2 shows:

A ThT-aggregation assay of tau was performed for quantifying the relative content of fibrils in the presence of peptides and heparin (6 µM). The concentration of tau-protein was 24 µM, the peptides were added at a ratio of 1:5 and 1:10 (tau-protein:peptide). The fluorescence measured of 24 µM tau-protein after incubation for 36h was arbitrarily set to be 100%, and the measured values and standard deviations of the other incubations are indicated as percentage proportions of this maximum value.

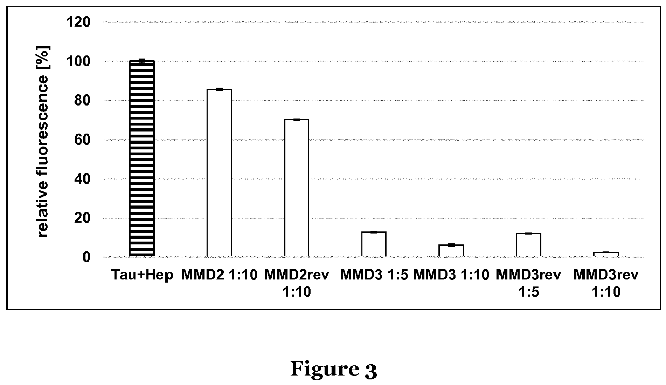

### Figure 3 shows:

A ThT-aggregation assay of tau was performed for quantifying the relative content of fibrils in the presence of peptides and heparin (6 µM). Here, the D-enantiomers of the peptides and their retro-inverso-versions are used. "retro-inverso-version" as used herein, refers to a sequence wherein the amino acid sequence of one peptide, as read from its N-terminus to its C-terminus, has been reversed, such that the N-terminus and C-terminus of the peptide sequence have been inverted. The concentration of tau-protein was 24 µM, and the peptides MMD2 and MMD2rev were added at a ratio of 1:10. The peptides MMD3 and MMD3rev were added at a ratio of 1:5 and 1:10 (tau-protein:peptide). The fluorescence of tau-protein at 24 µM after an incubation of 36 h was arbitrarily set to be 100% and the measured values and standard deviations of the other incubations are indicated as percentage proportions of this maximum value.

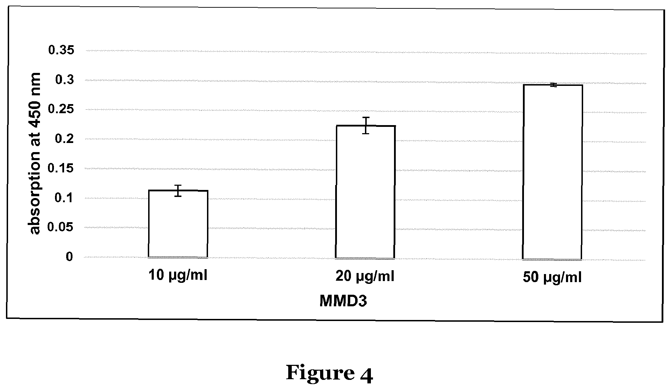

### Figure 4 shows:

An ELISA was performed to demonstrate binding of peptide MMD3-Lys(FAM)-NH2 to PHF6*-fibrils. As used herein, "PHF6*-fibrils" are fibrils comprising the D-enantiomeric version of tau-peptide VQIINK (SEQ ID NO: 5). The wells of a 96-well plate were coated with 50 µg/ml PHF6*-fibrils and were incubated for 1h. After washing of wells three times with PBS-T (phosphate buffer saline with 0.1 % tween), the free binding sites were blocked for 1 hour. Peptide MMD3-Lys(FAM)-NH2 was added (10 µg/ml, 20 µg/ml and 50 µg/ml) and incubated for 1 h. A specific anti-FITC-HRP-antibody was added at a dilution of 1:1000 and incubated for 1h. Thereafter, TMB-substrate solution was added and incubated for 20 min. After termination of the enzyme reaction by additional 20 % sulfuric acid, absorption at 450 nm was measured. The absorption of the negative control (buffer only) was substracted. Triplicate measurements were performed, at the average values of the measured absorptions of the different samples are shown as well as their respective standard deviations.

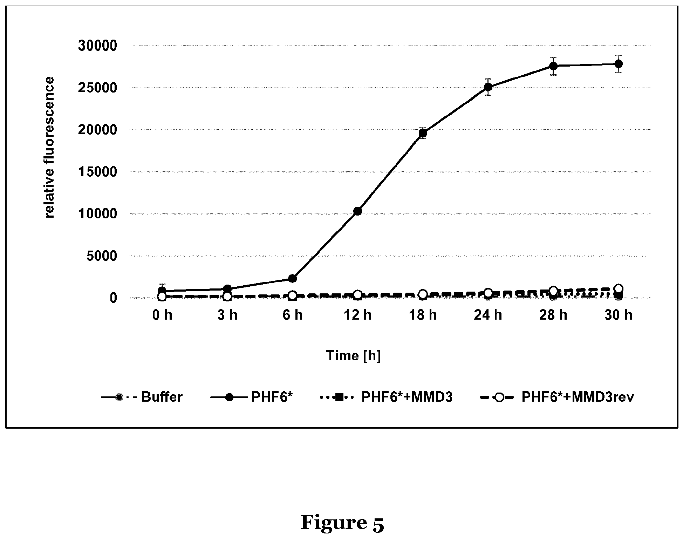

### Figure 5 shows:

Thioflavin T-tests were performed to demonstrate that peptides MMD3 and MMD3rev inhibit the aggregation of PHF6*-fibrils. PHF6*-fibrils are fibrils comprising the D-enantiomeric version of tau-peptide VQIINK (SEQ ID NO: 5). Peptides MMD3 and MMD3rev were diluted in sodium phosphate buffer to 1000 µM, and 100 µM PHF6*-solution and 10 µM thioflavin T were added. 100 µM PHF6* and 10 µM thioflavin in Napi-buffer was used as positive control. The negative control was 10 µM thioflavin T in Napi-buffer only. The samples were pipetted into a black 96-well µclear flat-bottom plate and incubated for 30 h,the relative fluorescence (excitation = 440 nm and emission = 480 nm) was determined. Triplicate measurements were performed for each sample. Figure 5 shows the time in hours on the x-axis and the average values of measured relative fluorescences of the different samples as well as their respective standard deviations on the y-axis.

Moreover, reference is made to the following examples which are given to illustrate and not to limit the present invention.

### Examples

### Example 1

### Binding Assay of Peptides to Monomeric Tau-Protein and Tau-Fibrils

The binding affinity of MMD2 and MMD3 was tested using ELISA. After coating a 96-well plate with 20 µg/ml tau monomers as well as tau fibrils, the FAM-labeled peptides MMD2-FAM and MMD3-FAM were added in increasing concentrations. The detection was done with anti-FAM HRP antibodies and absorbance was measured at 450 nm. As negative control, only coating buffer was incubated in the wells.

For MMD2 and MMD3 binding could be demonstrated for tau monomers as well as for tau fibrils. No significant difference could be detected between the binding affinities of both peptides to the different conformers with a minimal preference for tau monomers. The binding affinity of MMD2 to tau monomers and tau fibrils seemed to be stronger than MMD3.

### Example 2

### ThT-Aggregation Assay

To test the ability of the L-synthesized peptides to inhibit the aggregation of tau in vitro, thioflavin assays were performed. The peptides were added to the aggregation mix in a molar ratio of 1:5 and/or 1:10 (tau:peptide), respectively. As positive control, tau was incubated with heparin without addition of peptides, negative controls were tau alone with THT and buffer including THT. The relative fluorescence of the positive control was set to be 100% after 36 h of incubation and the relative fluorescence of the samples was normalized to the positive control. As figure 2 shows, MM2 and MM3 reduced tau fibril formation significantly, especially in a ratio of 1:10. MM2 reduced the aggregation of tau to about 50% in molar ratio of 1:5, in molar ratio 1:10 reached MM2 nearly complete inhibition of tau fibrils formation. In case of MM3, both molar ratios 1:5 and 1.10 were nearly equally efficient in significantly inhibiting tau fibrils formation.

To investigate the potential of the selected D-peptides MMD2, MMD2rev, MMD3 and MMD3rev to inhibit fibrillization of full-length tau, thioflavin fluorescence assays were performed. Tau aggregation was initiated by addition heparin to tau protein in a concentration of 24 µM, the mixtures were incubated in absence or presence of the respective peptide in a molar ratio of 1:10, in case of MMD2 as well as MMD2rev, and in molar ratio 1:10 and 1:5, in case of MMD3 and MMD3rev. After 36 hours of incubation, the fluorescence of the tau and heparin control sample reached a saturation level, and the value of fluorescence was set to 100%. As can be seen in figure 3, both MMD2 and MMD2rev did not inhibit the aggregation of tau significantly in molar ratio of 1:10 (tau:peptide). MMD2 reduce the aggregation of tau to about 90% and MMD2rev reduce the aggregation to about 70%. However, in contrast thereto, both peptides MMD3 and MMD3rev inhibited formation of tau aggregates significantly, in particular in molar ratio of 1:10 and 1:5. The inventors observed also that ThT fluorescence decreased with increasing concentration of the MMD3 and MMD3rev signifying a robust dose-dependent inhibition of full-length tau aggregation. MMD3 and MMD3rev were equally efficient in significantly inhibiting tau fibrils formation.

### Example 3

### Binding to PHF6*-Fibrils

To evaluate the binding properties of MMD3 to PHF6* fibrils, ELISA was performed with FAM-labeled version of the peptide. The plate was coated with 50 µg/ml PHF6* fibrils, as negative control, only coating buffer was added to the wells. The peptide was added in an increasing concentration, the bound peptide was detected with anti-FAM antibodies. After adding the substrate, the absorption at 450 nm, which represents the binding affinities, was measured. As shown in figure 4, MMD3 bound to PHF6* fibrils. The inventors observed that the absorption signal increased with increasing the concentration of MMD3, which indicates that MMD3 bound to PHF6* fibrils in concentration-dependent manner.

### Example 4

### Inhibition of PHF6*-Aggregation in the Presence of Peptides

To test whether MMD3 and MMD3rev reduce PHF6* fibril formation, 1000 µM from the respective peptides were incubated with 100 µM PHF6* at RT, as positive control PHF6* was incubated alone. The relative fluorescence of THT was monitored over 30 h. As expected, MMD3 and MMD3rev, which had been selected against PHF6* fibrils, did indeed inhibit the formation of PHF6* fibrils.

## Claims

1. A peptide binding to tau-protein, or tau-protein fragments, said peptide comprising an amino acid sequence selected from
DPLKARHTSVWY (SEQ ID NO: 1), and
YWVSTHRAKLPD (SEQ ID NO: 2) and dimers or multimers of peptides comprising such sequence(s), and homologs, derivates or fragments of peptides comprising such sequence(s).

2. The peptide according to claim 1, consisting of an amino acid selected from
DPLKARHTSVWY (SEQ ID NO: 1), and
YWVSTHRAKLPD (SEQ ID NO: 2).

3. The peptide according to any of the foregoing claims, wherein said amino acid sequence is made up of L-amino acid residues or D-amino acid residues, or a combination thereof.

4. The peptide according to any of the foregoing claims, wherein said amino acid sequence is selected from
DPLKARHTSVWY (SEQ ID NO: 1),
YWVSTHRAKLPD (SEQ ID NO: 2),
dplkarhtsvwy (SEQ ID NO: 3), and
ywvsthraklpd (SEQ ID NO: 4),
wherein upper case letters represent L-amino acid residues or D-amino acid residues, preferably L-amino acid residues, and lower case letters represent D-amino acid residues.

5. The peptide according to any of claims 1, 3-4, further comprising a tag attached to said peptide to facilitate detection or purification of the peptide, or to increase solubility thereof, or to increase blood-brain-barrier permeability; or further comprising a tag attached that is directed at A-beta-protein.

6. The peptide according to any of the foregoing claims, wherein said peptide binds to tau-monomers, oligomers or -fibrils , in particular to tau-oligomers or tau-fibrils comprising a peptide sequence segment having the sequence VQIINK (SEQ ID NO: 5).

7. The peptide according to any of the foregoing claims, wherein said peptide inhibits aggregation of monomeric tau-protein and/or aggregation or extension of tau-oligomers or tau-fibrils.

8. A pharmaceutical composition comprising a peptide according to any of the foregoing claims and a pharmaceutically acceptable excipient.

9. The peptide according to any of claims 1-7 or the pharmaceutical composition according to claim 8, for use in method of treating and/or preventing a tauopathy, wherein preferably said tauopathy is selected from Alzheimer's disease (AD), primary age-related tauopathy, frontotemporal dementia, chronic traumatic encephalopathy (CTE), progressive supranuclear palsy, corticobasal degeneration, Lytico-bodig disease, ganglioglioma, gangliocytoma, Meningioangiomatosis, postencephalitic parkinsonism, and subacute sclerosing panencephalitis.

10. The peptide or pharmaceutical composition for use according to claim 9, wherein said method comprises administering an effective amount of said peptide or of said composition to a patient in need thereof.

11. The peptide according to any of claims 1-7 or the pharmaceutical composition according to claim 8, for use in a method of diagnosing a tauopathy, wherein preferably said tauopathy is selected from Alzheimer's disease (AD), primary age-related tauopathy, frontotemporal dementia, chronic traumatic encephalopathy (CTE), progressive supranuclear palsy, corticobasal degeneration, Lytico-bodig disease, ganglioglioma, gangliocytoma, Meningioangiomatosis, postencephalitic parkinsonism, and subacute sclerosing panencephalitis.

12. The peptide or pharmaceutical composition for use according to claim 11, wherein said method comprises administering an effective amount of said peptide or of said composition to a subject to be tested for a tauopathy.

13. The peptide or pharmaceutical composition for use according to any of claims 11-12, wherein said peptide is linked to or administered together with a suitable reporter molecule that allows detection of monomeric tau-protein, tau-oligomers or of tau-fibrils and/or aggregates thereof, by a suitable detection methodology, such as positron emission tomography (PET), nuclear magnetic resonance (NMR), magnetic resonance imaging (MRI) and PET-MRI, and wherein said subject, after administration of said peptide or of said pharmaceutical composition, is subjected to said suitable detection methodology, such as PET, NMR, MRI, PET-MRI.

14. A kit for the in-vitro or in-vivo detection of tau-protein aggregates or tau-fibrils, or for the diagnosis of a tauopathy, said kit comprising the peptide according to any of claims 1-7, in a freeze-dried form in a suitable container, a buffered solvent in a separate container for reconstitution of said peptide in solution, and, optionally, means to dispense said peptide once reconstituted in solution, such as a syringe or pipette.

15. Use of the peptide according to any of claims 1-7, in an in-vitro assay, preferably in an in-vitro immunochemistry assay, such as an enzyme-linked immunosorbent assay (ELISA) or a radio-immuno assay (RIA).
